# EUROPEAN PATENT SPECIFICATION

(11) **EP 2 102 166 B1**
(45) Date of publication and mention of the grant of the patent: **18.07.2012**
(21) Application number: 07831970.4
(22) Date of filing: 09.11.2007
(51) Int. Cl.: C07D 231/38

(54) **PROCESS FOR PRODUCING 5-AMINOPYRAZOLE DERIVATIVE, AND AZO DYE**
VERFAHREN ZUR HERSTELLUNG EINES 5-AMINOPYRAZOLDERIVATS UND AZOFARBSTOFF
PROCÉDÉ DE PRODUCTION DE DÉRIVÉ DE 5-AMINOPYRAZOLE, ET COLORANT AZOÏQUE

(30) Priority: 10.11.2006 JP 2006304998; 10.11.2006 JP 2006304999; 15.08.2007 JP 2007211816
(43) Date of publication of application: 23.09.2009
(73) Proprietor: FUJIFILM Corporation, Tokyo 106-0031 (JP)
(72) Inventor: TATEISHI, Keiichi, Minami-Ashigara-shi, Kanagawa (JP); TAKASAKI, Masaru, Minami-Ashigara-shi, Kanagawa (JP); TANAKA, Shigeaki, Minami-Ashigara-shi, Kanagawa (JP); SEKIOKA, Tadao, Minami-Ashigara-shi, Kanagawa (JP); MORITA, Takahiro, Odawara-shi, Kanagawa (JP)
(74) Representative: HOFFMANN EITLE
(86) International application number: PCT/JP2007/072240
(87) International publication number: WO 2008/056828

(56) References cited:
- WO-A-03/076409
- WO-A-2006/018718
- WO-A-2006/072589
- WO-A-2006/082669
- WO-A-2006/108640
- NAM, N. L. ET AL: "Synthesis of N(1)-substituted 5-amino-3- methylpyrazoles" CHEMISTRY OF HETEROCYCLIC COMPOUNDS (NEW YORK)(TRANSLATION OF KHIMIYA GETEROTSIKLICHESKIKH SOEDINENII) , 36(3), 281-283 CODEN: CHCCAL; ISSN: 0009-3122, 2000, XP002468509
- DUMAS J ET AL: "1-Phenyl-5-pyrazolyl ureas: potent and selective p38 kinase inhibitors" BIOORGANIC & MEDICINAL CHEMISTRY LETTERS, OXFORD, GB, vol. 10, no. 18, September 2000 (2000-09), pages 2051-2054, XP004208309 ISSN: 0960-894X
- SURYAKIRAN ET AL: "An expeditious synthesis of 3-amino 2H-pyrazoles promoted by methanesulphonic acid under solvent and solvent free conditions" JOURNAL OF MOLECULAR CATALYSIS. A, CHEMICAL, ELSEVIER, AMSTERDAM, NL, vol. 258, no. 1-2, 2 October 2006 (2006-10-02), pages 371-375, XP005652144 ISSN: 1381-1169
- MARKWALDER ET AL: "Synthesis and Biological Evaluation of 1-Aryl-4,5-dihydro-1H- pyrazolo[3,4-d]pyrimidin-4-one Inhibitors of Cyclin-Dependent Kinases" JOURNAL OF MEDICINAL CHEMISTRY, AMERICAN CHEMICAL SOCIETY. WASHINGTON, US, vol. 47, no. 24, 2004, pages 5894-5911, XP002400752 ISSN: 0022-2623

## Description

### Technical Field

The present invention relates to a process for producing a 5-aminopyrazole derivative substituted with a water-soluble group. More specifically, the invention relates to a process for producing a 5-aminopyrazole derivative (coupler component) substituted with a hydroxyl group, carboxyl group, or sulfo group in an inexpensive water solvent at a high yield and at a high purity.

### Background Art

5-aminopyrazole derivatives are useful compounds as intermediate products for functional compounds such as photographic additives, sensitizing dyes, dyes, pigments, electronic materials, and medical and agricultural chemicals, and synthesis methods have been known long since (Gazz. chim. ital. 81, 380 (1951), Gazz. chim. ital. 82, 373 (1952), and "Chemistry for Heterocyclic Compound" (published on 1988-04-01) written by Hiroshi Yamanaka, et al.). Among the known synthesis methods, reaction of hydrazine compounds and a 1,3-dicarbonyl compounds such as β-ketoester or β-ketonitrile can be mentioned as typical and general purpose synthesis methods.

On the other hand, also for the synthesis method of 5-aminopyrazole derivatives (coupler component) substituted with water-soluble groups, they can be obtained by reaction between hydrazine compounds and β-ketonitrile. WO 06/082669 describes that they are reacted under a strongly acidic condition by using a mixed solvent of ethylene glycol and methanol. However, in a case of reaction under the strongly acidic condition, there is a problem that the purity is lowered since ethylene glycol is contained in crude crystals of obtained 5-aminopyrazole derivatives. Further, JP-A-2006-57076 describes reaction under an alkaline condition by using an aqueous solvent. However, the method involves a problem that the yield is extremely low since a great amount of by-products are also formed.

Since the hydrazine compound represented by Formula (II) is easily soluble to water in strongly acidic condition or alkaline condition, the reaction of the invention is usually conducted generally in a strongly acidic condition or neutral condition to alkaline condition. However, this involves a problem that the reaction per se does not proceed under the strongly acidic condition or the alkaline condition or by products are formed in a great amount depending on the type of the reaction system or the molecular structure of a reaction substrate and both the yield and the purity were not satisfactory. As described above, it has been found that the purity is low or the yield is low under the strongly acidic condition or the alkaline condition in a case of reaction with an aqueous solvent system which is inexpensive and gives less burden on the environment as a synthesis method of water-soluble group-substituted 5-amino pyrazole derivatives (coupler component).

Further information regarding the synthesis of 5-aminopyrazole derivatives can be found in Nam et al, Chemistry of Heterocyclic Compounds, Vol 36 (3), 2000, pages 281 - 283, WO 2006/072589, and WO 03/076409.

Further, a synthesis method, particularly, for the 5-amino-4-cyano pyrazole derivative (diazo component) includes reaction between a hydrazine compound and an alkoxymethylene malononitrile as a typical synthesis method (described in US-Pat-3336285). Further, JP-A-2006-057076 or WO 06/082669 describes examples of synthesis for obtaining 5-amino-4-cyanopyrazole derivatives substituted with carboxyl group using anilines substituted with a carboxyl group such as 5-aminoisophthalic acid as the starting material. However, this cannot always be said to be a production process which is simple and convenient and providing high yield and high purity since the yield is lowered due to the isolation loss and impurities are intruded inevitably because of extremely poor filterability to lower the purity in the step of isolation a hydrazine compound as an intermediate synthesis product. Further, since reaction of the obtained hydrazine compound and ethoxymethylene malononitrile is conducted in an organic solvent, this is a production process that gives a large burden on the environment with in view of liquid wastes, etc. As described above, in the process for producing 5-aminopyrazole derivative (diazo component) substituted with a water-soluble group, when the hydrazine compound substituted with water-soluble group formed as the intermediate synthesis product is isolated, the yield is lowered due to the isolation loss and the purity is lowered due to the worsening of the filterability and, further, the procedures are complicated to result in a problem of increasing the burden on the production.

### Disclosure of the Invention

Accordingly, the present invention intends to provide a process for producing 5-aminopyrazole derivative (coupler component) substituted with a water-soluble group represented by Formula (I) at a high yield and at a high purity in an aqueous system which is inexpensive and gentle to the environment.

As a result of a study for overcoming such problems in the related art, it has been found that using water which is inexpensive and gentle to the environment as a reaction solvent and starting the reaction at a pH upon charging within a range of from 2.0 to 4.0 (25°C) enable a 5-aminopyrazole derivative substituted with a water-soluble group represented by Formula (I) to be synthesized at a reduced cost and at a high yield and a high purity from the starting materials represented by Formula (II) and Formula (III).

That is, the foregoing objects of the invention have been attained by the method specified in claims 1-6.

"Upon charging" is defined herein as an instance the hydrazine compound represented by Formula (II), the compound represented by Formula (III), the reaction solvent, and the acidifying agent are mixed and stirred at 25°C for 5 min, that is, an instance the total reaction components are mixed and stirred at 25°C for 5 min.

### Best Mode for Carrying Out the Invention

Preferred embodiment of the invention is to be described specifically.

At first, the substituent in the present specification is to be described briefly. The substituent in the present specification includes the following groups (the groups are referred to as "substituent A").

For example, they include at halogen atom, alkyl group, alkenyl group, alkynyl group, allyl group, heterocyclic group, cyano group, hydroxyl group, nitro group, carboxyl group, alkoxy group, aryloxy group, sillyloxy group, heterocyclooxy group, acyloxy group, carbamoyloxy group, alkoxycarbonyloxy group, aryloxycarbonyloxy group, amino group, acylamino group, aminocarbonyl amino group, alkoxycarbonyl amino group, aryloxycarbonyl amino group, sulfamoyl amino group, alkyl or aryl sulfonyl amino group, mercapto group, alkylthio group, arylthio group, heterocyclic thio group, sulfamoyl group, sulfo group, alkyl or aryl sulfinyl group, alkyl or aryl sulfonyl group, acyl group, aryloxycarbonyl group, alkoxycarbonyl group, carbamoyl group, aryl or heterocyclic azo group, imido group, phosphino group, phosphinyl group, phosphinyloxy group, phosphinyl amino group, and silyl group.

The aliphatic group used in the present specification means an alkyl group, substituted alkyl group, alkenyl group, substituted alkenyl group, alkynyl group, and substituted alkynyl group. Further, the aromatic group used in the present specification means aryl group, and substituted aryl group.

More specifically, the halogen atom includes, for example, a fluorine atom, chlorine atom, bromine atom, and iodine atom.

The alkyl group includes linear, branched, or cyclic substituted or non-substituted alkyl groups and also includes cycloalkyl group, bicycloalkyl group, and, tricycle structure with more cyclic structure. The alkyl group in the substituent to be described later (for example, alkyl group in the alkoxy group or alkyl thio group) also represents the alkyl group of such meaning. Specifically, the alkyl group includes, preferably, alkyl groups of from 1 to 30 carbon atoms, for example, methyl group, ethyl group, n-propyl group, isopropyl group, t-butyl group, n-octyl group, eicosyl group, 2-chloroethyl group, 2-cyanoethyl group, and 2-ethylhexyl group, the cycloalkyl group includes, preferably, substituted or non-substituted cycloalkyl groups of 3 to 30 carbon atoms, for example, cyclohexyl group, cyclopentyl group, and 4-n-dodecylcycloalkyl group. The bicycloalkyl group includes, preferably, substituted or non-substituted bicycloalkyl groups having 5 to 30 carbon atoms, that is, this includes, for example, a monovalent group formed by removing one hydrogen atom from a bicycloalkane having 5 to 30 carbon atoms, for example, bicycle[1,2,2]heptane-2-yl group, bicycle[2,2,2]octane-3-yl group, etc.

The alkenyl group includes linear, branched or cyclic substituted or non-substituted alkenyl groups and include a cycloalkenyl group and bicycloalkenyl group. Specifically, the alkenyl group includes, preferably, substituted or non-substituted alkenyl groups of from 2 to 30 carbon atoms, for example, a vinyl group, aryl group, prenyl group, geranyl group, and oleyl group, the cycloalkenyl group includes, preferably, substituted or non-substituted cycloalkenyl groups of from 3 to 30 carbon atoms, that is, a monovalent group formed by removing one hydrogen atom from a cycloalkene having from 3 to 30 carbon atoms, for example, 2-cyclopentene-1-yl group, and 2-cyclohexene-1-yl group, and the bicycloalkenyl group includes substituted or non-substituted bicycloalkenyl groups, preferably, substituted or non-substituted bicyloalkenyl groups of from 5 to 30 carbon atoms, that is, a monovalent group formed by removing one hydrogen atom from a bicycloalkene having one double bond, for example, bicycle[2,2,1]hepto-2-en-1-yl group, bicycle[2,2,2]octo-2-en-4-yl group, etc.

The alkynyl group includes, preferably, substituted or non-substituted alkynyl groups of 2 to 30 carbon atoms, for example, ethynyl group, propargyl group, and trimethylsillylethynyl group.

The aryl group includes, preferably, substituted or non-substituted aryl groups of 6 to 30 carbon atoms, for example, phenyl group, p-tolyl group, naphthyl group, m-chlorophenyl group, and o-hexadenoyl aminophenyl group.

The heterocyclic group is, preferably, a monovalent group formed by removing one hydrogen atom from a 5- or 6-membered substituted or non-substituted aromatic or non-aromatic heterocyclic compound and, more preferably, a 5- or 6-membered heterocyclic group of 3 to 30 carbon atoms and may be either a single nuclear structure or a polynuclear structure in which two or more rings are condensated. Further, as the heterocyclic group, heterocyclic groups containing at least one of N, O, S atoms are preferred. They include, for example, thienyl group, furyl group, pyrrolyl group, indolyl group, imidazolyl group, benzimidazolyl group, pyrazolyl group, indozolyl group, thiazolyl group, benzothiazolyl group, isothiazolyl group, benzoisothiazolyl group, oxazolyl group, benzoxazolyl group, isooxazolyl group, 1,2,4-thiadiazolyl group, 1,3,4-thiadiazolyl group, 1,2,4-oxadiazolyl group, 1,3,4-oxadiazolyl group, triazolyl group, piridyl group, pirazyl group, pirimidyl group, piridazyl group, 1,3,5-triazyl group, quinolyl group, isoquinolyl group, and phthaladinyl group.

The alkoxy group includes, preferably, substituted or non-substituted alkoxy groups of 1 to 30 carbon atoms, for example, methoxy group, ethoxy group, isopropoxy group, t-butoxy group, n-octyloxy group, and 2-methoxyethoxyl group.

The aryloxy group includes, for example, substituted or non-substituted aryloxy groups of 6 to 30 carbon atoms, for example, phenoxy group, 2-methylphenoxy group, 4-t-butylphenoxy group, 3-nitrophenoxy group, and 2-tetradecanoylaminophenyl group.

The silyloxy group includes, for example, substituted or non-substituted silyloxy groups of 0 to 20 carbon atoms, for example, trimethylsilyloxy group and diphenylmethylsilyloxy group.

The heterocyclicoxy group includes, preferably, substituted or non-substituted heterocyclic oxy groups of 2 to 30 carbon atoms, for example, 1-phenyltetrazole-5-oxy group and 2-tetrahydropyranyloxy group.

Acyloxy group includes, preferably, substituted or non-substituted alkylcarbonyloxy groups of 2 to 30 carbon atoms, substituted or non-substituted aryl carbonyloxy group of 6 to 30 carbon atoms, for example, acetyloxy group, pivaloyl group, stearoyloxy group, benzoyloxy group, and p-methoxyphenyl carbonyloxy group. The carbamoyloxy group includes, preferably, substituted or non-substituted carbamoyloxy groups of 1 to 30 carbon atoms, for example, N,N-dimethylcarbamoyloxy group, N,N-diethylcarbamoyloxy group, morpholinocarbonyloxy group, N,N-di-n-octylaminocarbonyloxy group, and N-n-octylcarbamoyloxy group.

The alkoxycarbonyloxy group includes, preferably, substituted or non-substituted alkoxycarbonyloxy groups of 2 to 30 carbon atoms, for example, methoxycarbonyloxy group, ethoxycarbonyloxy group, t-butoxycarbonyloxy group, and n-octylcarbonyloxy group.

The aryloxycarbonyloxy group includes, preferably, substituted or non-substituted aryloxy carbonyloxy groups of 7 to 30 carbon atoms, for example, phenoxycarbonyloxy group, p-methoxyphenoxy carbonyloxy group, and p-n-hexadecyloxyphenoxy carbonyloxy group.

The amino group includes alkylamino groups, arylamino groups, heterocyclic amino groups and includes, preferably, amino group, substituted or non-substituted alkylamino groups of 1 to 30 carbon atoms, substituted or non-substituted anilino groups of 6 to 30 carbon atoms, for example, methylamno group, dimethylamino group, aniline group, N-methyl-anilino group, and diphenylamino group.

The acylamino group includes, preferably, formyl amino group, substituted or non-substituted alkylcarbonyl amino groups of 1 to 30 carbon atoms and substituted or non-substituted arylcarbonyl amino groups of 6 to 30 carbon atoms, for example, acetylamino group, pivaloylamino group, lauroylamino group, benzoylamino group, and 3,4,5-tri-n-octyloxyphenyl carbonylamikno group.

The aminocarbonylamino group includes, for example, substituted or non-substituted aminocarbonyl amino groups of 1 to 30 carbon atoms, for example, carbamoyl amino group, N,N-dimethylamino carbonylamino group, N,N-diethylamino carbonylamino group, and morpholino carbonylamino group.

The alkoxycarbonyl amino group includes, preferably, substituted or non-substituted alkoxycarbonylamino groups of 2 to 30 carbon atoms, for example, methoxycarbonylamino group, ethoxycarbonylamino group, t-butoxycarbonylamino group, n-octadecyloxy carbonylamino group, and N-methyl-methoxy carbonylamino group.

The aryloxy carbonylamino group includes, preferably, substituted or non-substituted aryloxy carbonylamino groups of 7 to 30 carbon atoms, for example, phenoxycarbonylamino group, p-chlorophenoxycarbonylamino group, and m-n-octyloxyphenoxy carbonylamino group.

The sulfamoylamino group includes, preferably, substituted or non-substituted sulfamonylamino groups of 0 to 30 carbon atoms, for example, sulfamolyamino group, N,N-dimethylamino sulfonylamino group, and N-n-octylamino sulfonylamino group.

The alkyl or aryl sulfonylamino group includes, preferably, substituted or non-substituted alkylsulfonylamino groups of 1 to 30 carbon atoms and substituted or non-substituted arylsulfonylamino groups of 6 to 30 carbon atom, for example, methylsulfonylamino group, butylsulfonylamino group, phenylsulfonylamino group, 2,3,5-trichlorophenyl sulfonylamino group, and p-methylphenyl sulfonylamino group.

The alkylthio group includes, preferably, substituted or non-substituted alkylthio groups of 1 to 30 carbon atoms, for example, methylthio group, ethylthio group, and n-hexadecylthio group.

The arylthio group includes, preferably, substituted or non-substituted arylthio groups of 6 to 30 carbon atoms, for example, phenylthio group, p-chlorophenylthio group, and m-methoxyphenylthio group.

The heterocyclic thio group includes, preferably, substituted or non-substituted heterocyclic thio groups of 2 to 30 carbon atoms, for example, 2-benzothizolyl group, and 1-phenyltetrazole-5-yl thio group.

The sulfamoyl group includes, for example, substituted or non-substituted sulfamoyl groups of 0 to 30 carbon atoms, for example, N-ethylsulfamoyl group, N-(3-dodecyloxypropyl)sulfamoyl group, N,N-dimethylsulfamoyl group, N-acetylsulfamoyl group, N-benzoylsulfamoyl group, and N-(N'-phenylcarbamoyl)sulfamoyl group.

The alkyl or arylsulfinyl group includes, preferably, substituted or non-substituted alkylsulfinyl groups of 1 to 30 carbon atoms and substituted or non-substituted arylsulfinyl groups of 6 to 30 carbon atoms, for example, methylsulfinyl group, ethylsulfinyl group, phenylsulfinyl group, and p-methylphenylsulfinyl group.

The alkyl or arylsulfonyl group includes, preferably, substituted or non-substituted alkylsulfonyl groups of 1 to 30 carbon atoms and substituted or non-substituted arylsulfonyl groups of 6 to 30 carbon atoms, for example, methylsulfonyl group, ethylsulfonyl group, phenylsulfonyl group, and p-methylphenylsulfonyl group.

The acyl group includes, preferably, formyl group, substituted or non-substituted alkylcarbonyl groups of 2 to 30 carbon atoms, substituted or non-substituted arylcarbonyl groups of 7 to 30 carbon atoms, substituted or non-substituted heterocyclic carbonyl groups of 2 to 30 carbon atoms bonded at the carbon atom with a carbonyl group, for example, acetyl group, pivaloyl group, 2-chloroacetyl group, stearolyl group, benzoyl group, p-n-octyloxyphenyl carbonyl group, 2-pyrizylcarbonyl group, and 2-furylcarbonyl group.

The aryloxycarbonyl group includes, preferably, substituted or non-substituted aryloxycarbonyl groups of 7 to 30 carbon atoms, for example, phenoxycarbonyl group, o-chlorophenoxycarbonyl group, m-nitrophenoxycarbonyl group, and p-t-butylphenoxycarbonyl group.

The alkoxycarbonyl group includes, preferably, substituted or non-substituted alkoxycarbonyl groups of 2 to 30 carbon atom, for example, methoxycarbonyl group, ethoxycarbonyl group, t-butoxycarbonyl group, and n-octadecyloxycarbonyl group.

The carbamoyl group includes, preferably, substituted or non-substituted carbamoyl groups of 1 to 30 carbon atoms, for example, carbamoyl group, N-methylcarbamoyl group, N,N-dimethylcarbamoyl group, N,N-di-n-octylcarbamoyl group, and N-(methylsulfonyl)carbamoyl group.

The aryl or heterocyclic azo group includes, preferably, substituted or non-substituted arylazo groups of 6 to 30 carbon atoms or substituted or non-substituted heterocyclic azo groups of 3 to 30 carbon atoms, for example, phenylazo, p-chlorophenyl azo, and 5-ethylthio-1,3,4-thiadiazole-2-yl azo.

The imido group includes, preferably, N-succineimide group, N-phthalimide group, etc.

The phosphino group includes, preferably, substituted or non-substituted phosphino groups of 0 to 30 carbon atoms, for example, dimethylphosphino group, diphenylphosphino group, and methylphenoxyphosphino group.

The phosphinyl group includes, preferably, substituted or non-substituted phosphinyl groups of 0 to 30 carbon atoms, for example, phosphinyl group, dioctyloxyphosphinyl group, and diethoxyphosphinyl group.

The phosphinyloxy group includes, preferably, substituted or non-substituted phosphinyloxy groups of 0 to 30 carbon atoms, for example, diphenylxyphosphinyloxy group and dioctyloxyphosphinyloxy group.

The phosphinylamino group includes, preferably, substituted or non-substituted phosphinylamino groups of 0 to 30 carbon atoms, for example, dimethoxyphosphinylamino group, and dimethylaminophosphinylamino group.

The silyl group includes, preferably, substituted or non-substituted silyl groups of 0 to 30 carbon atoms, trimethylsilyl group, t-butyldimethylsilyl group, and phenyldimethylsilyl group.

Among the substituents described above, those having hydrogen atoms may be substituted at the hydrogen atoms by the substituents described above. Examples of such substituents include, alkylcarbonylaminosulfonyl group, arylcarbonylaminosulfonyl group, alkylsulfonyl aminocarbonyl group, and arylsulfonylaminocarbonyl group. The examples include methylsulfonylaminocarbonyl group, p-methylphenylsulfonylaminocarbonyl group, acetylamino sulfonyl group, and benzoylaminosulfonyl group.

R¹² represented by Formula (I) and Formula (III) represents a substituted or non-substituted aliphatic group, substituted or non-substituted aromatic group, or substituted or non-substituted heterocyclic group. The substituent includes the substituent A described above as an example. R¹² includes, preferably, non-substituted aliphatic groups, more preferably, alkyl groups of 5 or less carbon atoms and, particularly preferably, methyl group or tert-butyl group.

R¹¹ represented by Formula (I) and Formula (II) is a hydroxyl group, carboxyl group, or sulfo-substituted aromatic group (examples including those examples of the aryl groups described for the substituent A. They include preferably a phenyl group or naphthyl group) or heterocyclic group (examples including those examples of the heterocyclic groups described for the substituent A, preferably, pyridyl group, pyrimidyl group, 1,3,5-triazyl group). R¹¹ is, preferably, a heterocyclic group substituted with a hydroxyl group or a carboxyl group and, particularly preferably, hydroxyl group-substituted nitrogen-containing heterocyclic ring.

A particularly preferred structure of the 5-aminopyrazole derivative represented by Formula (I) is a compound represented by Formula (IV).

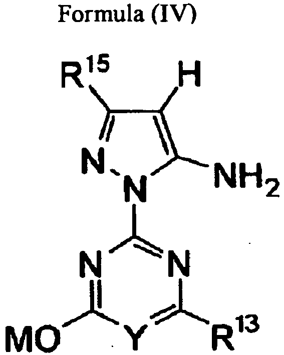

In Formula (IV), R¹³ represents a hydrogen atom or a substituent, Y represents a nitrogen atom or -CR¹⁴=, R¹⁵ has the same meanings as those for R¹² of Formula (I), and M represents a hydrogen atom, a metal ion, ammonium, or an organic cation. R¹⁴ represents herein a hydrogen atom or a substituent. In the formula, OM includes forms other than OH (keto-enol tautomerism), which corresponds to "hydroxyl group" in Formula (I).

R¹³ is, preferably, a hydrogen atom, halogen atom, alkyl group, heterocyclic group, hydroxyl group, alkoxy group, amino group, acylamino group, sulfamoylamino group, alkyl or arylsulfonylamino group, mercapto group, alkylthio or arylthio group, and particularly preferably, a hydrogen atom, hydroxyl group, halogen atom, amino group or heterocyclic group of 0 to 8 carbon atoms.

In Formula (IV), Y represents a nitrogen atom or -CR¹⁴=, and Y is preferably a nitrogen atom. In a case where Y is -CR¹⁴ =, R¹⁴ is preferably a hydrogen atom, halogen atom, alkyl group of 1 to 8 carbon atoms, alkoxy group of 1 to 8 carbon atoms, or a hydroxyl group.

In Formula (IV), M represents a hydrogen atom, a metal ion, ammonium, or an organic cation and, preferably, a hydrogen atom, an alkali metal ion (for example, lithium ion, sodium ion, potassium ion, cesium ion, etc.), an alkaline earth metal ion (for example, magnesium ion , calcium ion, etc.), silver ion, zinc ion, ammonium, quaternary ammonium (for example, tetraethyl ammonium, tetra-N-heptyl ammonium, dimethylcetyl benzyl ammonium, etc.) or quaternary phosphonium. More preferably, this is a hydrogen atom or an alkali metal ion, or ammonium.

In Formula (I) and Formula (IV), as the substituents for R¹¹ to R¹⁵, the substituent may join to each other to form a bis type, tris type or tetrakis type. R¹³ and R¹⁴ may join to each other to form a cyclic structure.

Then, preferred example of Formula (I) and Formula (IV) are mentioned as specific examples, but the invention is not restricted to them.

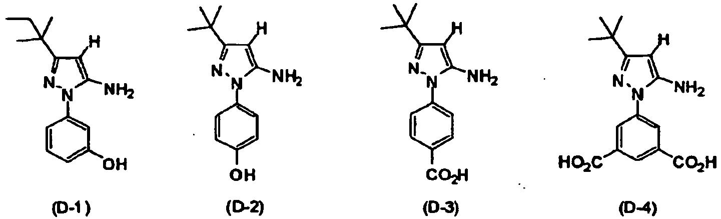

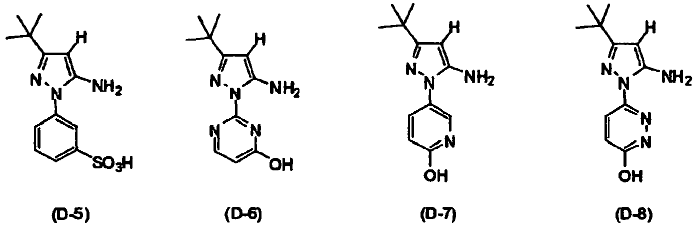

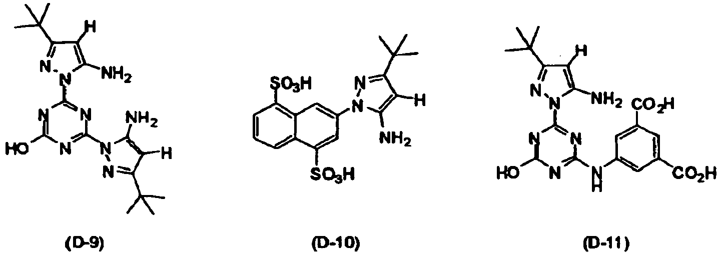

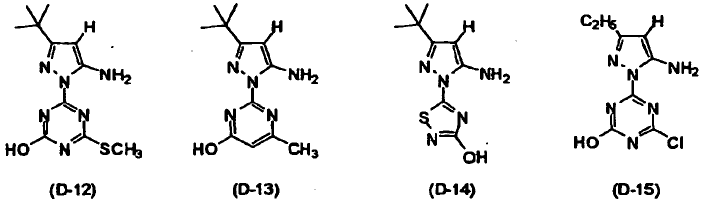

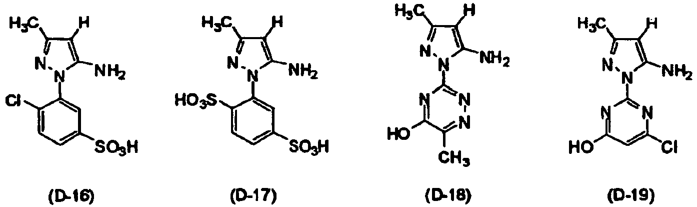

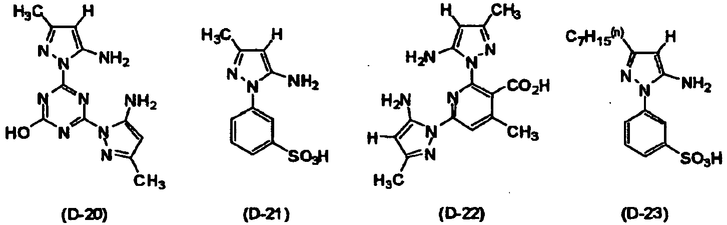

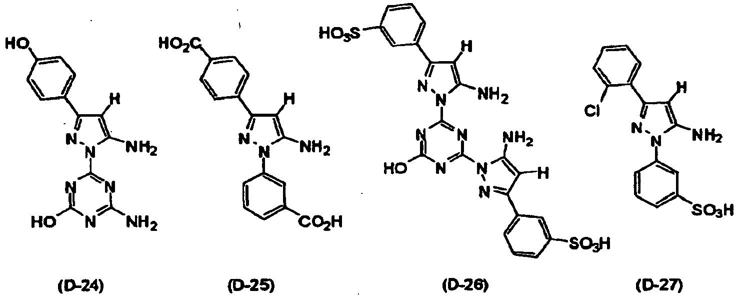

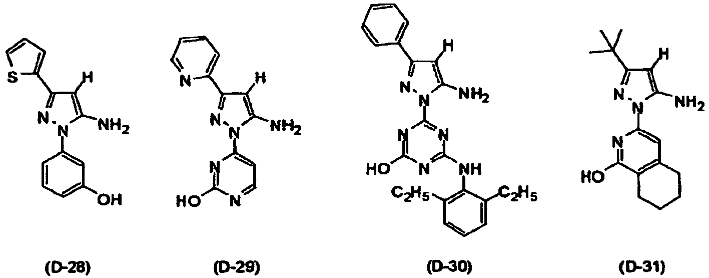

The compound described above of the invention may be present sometimes as a tautometric isomer depending on the type of the substituent. Both of optional tautomeric isomers in a pure form and an optional mixture of tautomeric isomers are included in the compound of the invention.

Further, in the invention, the compound represented by Formula (I) to Formula (IV) may contain isotopes (for example, ²H, ³H, ¹³C, ¹⁵N) in the structure.

The compound of the invention includes also those containing pair salts depending on the synthesis process or isolation method thereof. The pair salt may be of any type and includes, for example, halide ion, sulfate ion, nitrate ion, carbonate ion, sulfonate ion, phosphate ion, acetate ion, metal ion, and ammonium ion. Depending on the structure, it may form an intra-molecular salt.

Then, a process for producing the 5-aminopyrazole derivative represented by Formula (I) or Formula (IV) from the hydrazine compound represented by Formula (II) and the compound represented by Formula (III) of the invention is to be described specifically.

The production process of the invention has a feature of reacting the hydrazine compound represented by Formula (II) and the compound represented by Formula (III) under the reaction condition satisfying Reaction Condition 1 and the Reaction Condition 2;

### Reaction Condition 1: The reaction is conducted in a reaction solvent that consists of water or is a mixed solvent including 50% by mass or more water and an organic solvent.

Reaction Condition 2: pH of a liquid mixture containing the hydrazine compound represented by Formula (II), the compound represented by Formula (III), the reaction solvent, and an acidifying agent upon charging is within a range of from 2.0 to 4.0 at 25°C. In the production process of the invention, for adjusting the pH of the liquid mixture upon charging within a range from 2.0 to 4.0, it is necessary to add an acid or alkali. However, in a case where the pH of the liquid mixture of the hydrazine compound represented by Formula (II), the compound represented by Formula (III), and the reaction solvent upon charging is within a range from 2.0 to 4.0 (25°C), addition of the acid or alkali is not always necessary.

The acidifying agent means an acid for adjusting the pH.

The acid for adjusting the pH includes, for example, inorganic acids such as hydrochloric acid, hydrobromic acid, hydroiodic acid, sulfuric acid, nitric acid, phosphoric acid, and boric acid, or organic acids such as formic acid, acetic acid, trichloroacetic acid, propionic acid, methanesulfonic acid, and p-toluene sulfonic acid. The acid for adjusting the pH is preferably, hydrochloric acid, sulfuric acid, phosphoric acid, or methanesulfonic acid and, particularly preferably, sulfuric acid and hydrochloric acid.

The alkali for adjusting the pH may be an organic base (for example, ammonia, triethylamine, pyridine, etc.) or an inorganic base. The inorganic base used for adjusting the pH includes, for example, sodium hydrogen carbonate, potassium hydrogen carbonate, sodium carbonate, potassium carbonate, sodium acetate, potassium acetate, lithium hydroxide, sodium hydroxide, potassium hydroxide, monohydrogen sodium phosphate, dihydrogen sodium phosphate, and sodium phosphate and the alkali for adjusting the pH is, preferably, sodium hydroxide or potassium hydroxide.

The addition amount of the acid or alkali for adjusting the pH can be selected properly, and it is essential that the pH upon charging a liquid mixture containing the hydrazine compound represented by Formula (II), the compound represented by Formula (III), the reaction solvent, and the acid or alkali is from 2.0 to 4.0 (25°C) and the pH is preferably from 2.5 to 3.5 (25°C) and pH is, more preferably, from 2.7 to 3.2 (25°C).

Further, the pH upon completion of the reaction is preferably within a range from 0.8 to 2.0.

In the production of the compound of Formula (I) or Formula (IV), the reaction proceeds by using a solvent consisting only of water or a mixed solvent including water and an organic solvent. However, in a case of using a mixed solvent, the water content in the total mass of the reaction solvent is 50 mass% or more, the water content is preferably, 80 mass% or more and, particularly preferably, this is a solvent consisting only of water in view of production at a low cost.

The type of the organic solvent can be selected properly depending on the type of the reaction system or the like and can include, for example, acetonitrile, propionitrile, butylonitrile, alcohol (for example, methanol, ethanol, i-propylalcohol, ethylene glycol, etc), methylene chloride, chloroform, carbon tetrachloride, dichloroethane, ethyl acetate, butyl acetate, toluene, benzene, hexane, diethyl ether, tetrahydrofuran, 1,4-dioxane, N,N-dimethylformamide (DMF), N,N-dimethylacetoamide (DMAC), N,N-dimethylimidazolidinone (DMI), or sulfolane. The solvents may be combined properly and used as a mixture. The organic solvent in the reaction of the invention is, preferably, acetonitrile or alcohol and, particularly preferably, methanol or ethanol.

Further, while the amount of the solvent to be used is not particularly restricted and can be selected properly in accordance with the type of the reaction system or the like, it is usually about from 1 to 100 times, preferably, from 5 to 50 times and, particularly preferably, from 10 to 30 times by mass ratio of the solvent based on the hydrazine compound of Formula (II).

The reaction temperature in the invention is not particularly restricted and can be selected properly in accordance with the type of the reaction system and the concentration of the compounds of reaction species and it is usually about from 0°C to 120°C, preferably, from 20°C to 80°C, more preferably, from 30°C to 70°C and, particularly preferably, from 40°C to 60°C. While also the reaction time is not particularly restricted, it is usually from 30 min to 24 hr, from 2 hr to 15 hr and, particularly preferably, from 4 hr to 12 hr.

The order of adding the hydrazine compound represented by Formula (II), the compound represented by Formula (III), the reaction solvent, and the acid or the alkali into the reaction system is optional and not restricted particularly.

A synthesis scheme of an exemplified compound D-9 as an example of the 5-aminopyrazole derivative substituted with the water-soluble group represented by Formula (I) or Formula (IV) is shown below.

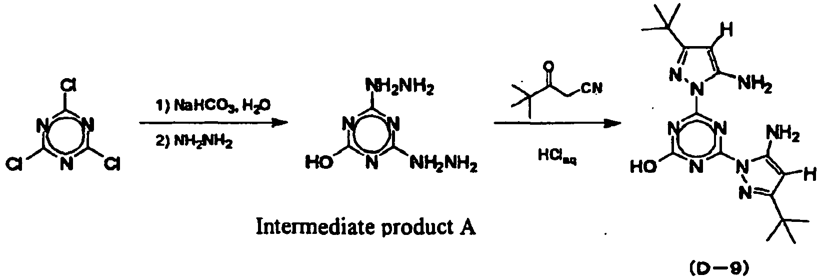

### Embodiment

Then, the invention is to be described more specifically by way of examples.

### [Example 1]

### < Production Process for Exemplified Compound (D-9) of Formula (I) >

### (I) Production Process for Intermediate Product A

An intermediate product A was synthesized in accordance with a method described in JP-A No. 2006-57076.

### (2) Production Process for Exemplified Compound (D-9)

500 mL of water was added to 20.0 g (127 mmmol) of an intermediate product A, and 34.9 g (279 mmmol) ofpivaloylacetonitrile, they were stirred at a room temperature (internal temperature: 24°C, pH: 5.08), and 56 mL (56 mmmol) of an aqueous 1 M solution of hydrochloric acid was added to a liquid reaction mixture (internal temperature: 25°C, pH upon charging: pH 2.81). After stirring at an internal temperature of 50°C for 8 hr (internal temperature: 50°C, pH: 1.20), when 27 to 28 mL of an aqueous 8 M solution of potassium hydroxide was dropped slowly, the reaction solution formed a homogenous system (internal temperature: 50°C, pH: 8.7 to 8.9). Further, after stirring for 30 min at an internal temperature of 50°C, when 13.5 mL of an aqueous 12 M solution of hydrochloric acid was dropped slowly, crystals were precipitated (internal temperature: 50°C, pH: 6.5 to 6.7). The crystals were collected by filtration and rinsed with 400 mL of water and dried to obtain 40.2 g of Exemplified Compound (D-9) (separation yield: 85.1%, HPLC purity: 97.1%).
¹H-NMR (400 MHz, d6-DMSO) 5.32 ppm (s, 2H), 1.21 ppm (s, 18H)

### (Comparative Example 1)

A synthesis method for the 5-aminopyrazole (cl) from the hydrazine compound (b1) is described in JP-A 2006-057076. However, although a water solvent is used, since the reaction is conducted under an alkaline condition, the separation yield was as low as 15%.

### [Example 2]

Then, reaction was conducted while changing the addition amount of hydrochloric acid under various pH conditions.

In accordance with the following experimental examples, the residual rate of the intermediate product A and the yield of formation of the Exemplified Compound (D-9) were determined according to the area (%) at 254 nm by HPLC measurement.

### < Experimental Example >

Water was added by X (mL) to 1.0 g (6.28 mmmol) of an intermediate product A and 1.75 g (14.0 mmmol) of pivaloylacetonitrile, they were stirred at a room temperature, and Y (mL) of an aqueous 12M solution of hydrochloric acid was added to the mixed solution (internal temperature: 25°C, result of pH measurement described in Table 1). The reaction solution after 4, 8, 10 hr was sampled at an internal temperature of 50°C and the residual rate of the intermediate product A and the yield of formation of Exemplified Compound (D-9) were determined according to the measurement HPLC.

**Table 1**

| | X (mL) | Y (mL) | pH upon charging | Reaction time | HPLC area (%) | | By-products |
|---|---|---|---|---|---|---|---|
| | | | | | Intermediate product A | (D-9) | |
| Comp. Example 1 | 20 | 1.63 | 0.48 | 4h | 16% | 66% | 18% |
| | | | | 10h | About equal with that for 4h | | |
| Comp. Example 2 | 20 | 0.70 | 1.45 | 4h | 8% | 76% | 16% |
| | | | | 10h | About equal with that for 4h | | |
| Invention 1 | 25 | 0.23 | 2.82 | 4h | 18% | 75% | 7% |
| | | | | 10h | 3% | 92% | 7% |
| Invention 2 | 25 | 0.12 | 3.04 | 4h | 22% | 72% | 5% |
| | | | | 8h | 3% | 96% | 1% |
| Comp. Example 3 | 20 | 0.05 | 4.50 | 4h | - | Trace amount | |
| | | | | 8h | 55% | 5% | 40% |

As shown in the table 1 described above it can be seen that, in a case where the pH upon charging the liquid mixture containing the hydrazine compound represented by Formula(II), the compound represented by Formula (III), the reaction solvent, and the acidifying agent is within a range from 2.5 to 3.0 (25°C), while the reaction rate lowers somewhat the yield of formation of the Exemplified Compound (D-9) is improved. In comparative examples 1, 2, by-products are 10% or more and the yield of formation of (D-9) is not improved even by the reaction for 10 hr.

The 5-aminopyrazole derivative substituted by the water-soluble group represented by Formula (I) corresponds to (cl) in ka 16 on page 27 of JP-A No. 2006-57076, which is useful as an intermediate synthesis product for dyes, etc. represented as (Dye 62) of the patent publication due to (5) or the like described on page 28 thereof.

### Industrial Applicability

According to the production process of the invention, the 5-aminopyrazole derivative (coupler component) substituted with the water-soluble group represented by Formula (1) is obtained at a high yield and a high purity in an aqueous system which is inexpensive and gentle to the environment.

## Claims

1. A process for producing a 5-aminopyrazole derivative represented by Formula (I), comprising:
a step of reacting a hydrazine compound represented by Formula (II) with a compound represented by Formula (III) under a condition satisfying Reaction Condition 1 and Reaction Condition 2, wherein
Reaction Condition 1 is that the reaction is carried out in a reaction solvent that consists of water or is a mixed solvent including 50 mass% or more water and an organic solvent,
Reaction Condition 2 is that pH of a liquid mixture containing the hydrazine. compound represented by Formula (II), the compound represented by Formula (III), the reaction solvent, and an acidifying agent upon charging is within a range of from 2.0 to 4.0 at 25°C: 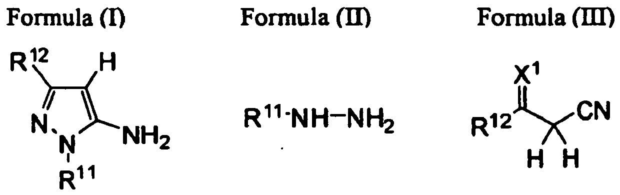 wherein
R¹¹ is a hydroxyl group-, carboxyl group-, or sulfo group-substituted aromatic group
R¹² represents an aliphatic group, aromatic group, or heterocyclic group each or heterocyclic group ;
of which is substituted or unsubstituted, and
X¹ represents an oxygen atom or NH.

2. The production process according to claim 1, wherein
R¹¹ in Formula (I) and Formula (II) is a heterocyclic group substituted with a hydroxyl group.

3. The production process according to claim 1 or 2, wherein the acidifying agent is sulfuric acid or hydrochloric acid.

4. The production process according to any of claims 1 to 3, wherein the reaction is conducted at a temperature of from 30 to 70°C.

5. The production process according to any of claims 1 to 4, wherein the reaction solvent consists only of water.

6. The production process according to any of claims 1 to 5, wherein
pH of the liquid mixture upon charging is within a range of from 2.5 to 3.5 at 25°C.

## Patentansprüche

1. Verfahren zur Herstellung eines durch die Formel (I) dargestellten 5-Aminopyrazolderivats, umfassend:
einen Schritt zum Umsetzen einer durch die Formel (II) dargestellten Hydrazinverbindung mit einer durch die Formel (III) dargestellten Verbindung unter Bedingungen, die Reaktionsbedingung 1 und Reaktionsbedingung 2 erfüllen, worin
Reaktionsbedingung 1 ist, dass die Reaktion in einem Reaktionslösungsmittel durchgeführt wird, das aus Wasser besteht oder das ein gemischte Lösungsmittel ist, das 50 Masse-% oder mehr Wasser und ein organisches Lösungsmittel enthält,
Reaktionsbedingung 2 ist, dass der pH-Wert einer flüssigen Mischung, die die durch die Formel (II) dargestellte Hydrazinverbindung, die durch die Formel (III) dargestellte Verbindung, das Reaktionslösungsmittel und ein Ansäuerungsmittel enthält, beim Einbringen innerhalb des Bereichs von 2,0 bis 4,0 bei 25°C liegt: 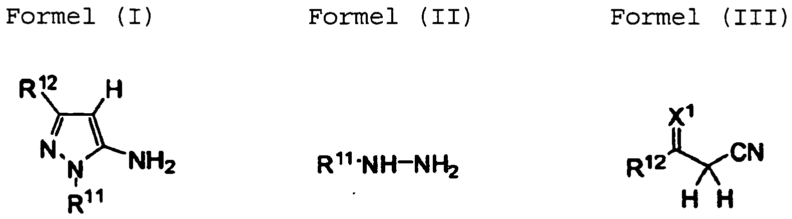 worin
R¹¹ eine Hydroxylgruppe-, Carboxylgruppe- oder Sulfogruppe-substituierte aromatische Gruppe oder heterocyclische Gruppe ist;
R¹² eine aliphatische Gruppe, aromatische Gruppe oder heterocyclische Gruppe darstellt, von denen jede substituiert oder unsubstituiert ist, und
X¹ ein Sauerstoffatom oder NH darstellt.

2. Herstellungsverfahren gemäß Anspruch 1, worin R¹¹ in Formel (I) und Formel (II) eine heterocyclische Gruppe ist, die mit einer Hydroxylgruppe substituiert ist.

3. Herstellungsverfahren gemäß Anspruch 1 oder 2, worin das Ansäuerungsmittel Schwefelsäure oder Salzsäure ist.

4. Herstellungsverfahren gemäß irgendeinem der Ansprüche 1 bis 3, worin die Reaktion bei einer Temperatur von 30 bis 70°C durchgeführt wird.

5. Herstellungsverfahren gemäß irgendeinem der Ansprüche 1 bis 4, worin das Reaktionslösungsmittel nur aus Wasser besteht.

6. Herstellungsverfahren gemäß irgendeinem der Ansprüche 1 bis 5, worin der pH-Wert der flüssigen Mischung beim Einbringen innerhalb eines Bereichs von 2,5 bis 3,5 bei 25°C liegt.

## Revendications

1. Procédé de production d'un dérivé de 5-aminopyrazole représenté par la formule (I), comprenant :
une étape de réaction d'un composé d'hydrazine représenté par la formule (II) avec un composé représenté par la formule (III) sous une condition satisfaisant la condition de réaction 1 et la condition de réaction 2, dans lequel
la condition de réaction 1 consiste en ce que la réaction est réalisée dans un solvant de réaction qui est constitué d'eau ou qui est un solvant mixte comprenant 50 % en masse ou plus d'eau et un solvant organique,
la condition de réaction 2 consiste en ce que le pH d'un mélange liquide contenant le composé d'hydrazine représenté par la formule (II), le composé représenté par la formule (III), le solvant de réaction, et un agent acidifiant se trouve lors de la charge dans un intervalle de 2,0 à 4,0 à 25°C : 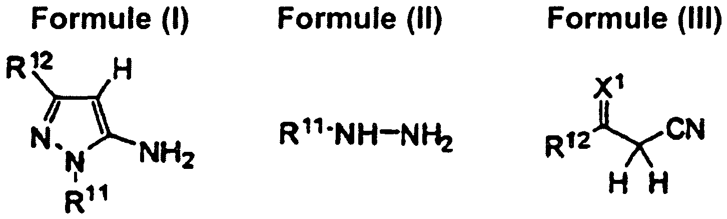 dans lesquelles
R¹¹ est un groupe aromatique ou un groupe hétérocyclique substitué par un groupe hydroxyle, un groupe carboxyle, ou un groupe sulfo;
R¹² représente un groupe aliphatique, un groupe aromatique, ou un groupe hétérocyclique dont chacun est substitué ou non substitué, et
X¹ représente un atome d'oxygène ou NH.

2. Procédé de production selon la revendication 1, dans lequel
R¹¹ dans la formule (I) et la formule (II) est un groupe hétérocyclique substitué par un groupe hydroxyle.

3. Procédé de production selon la revendication 1 ou 2, dans lequel l'agent acidifiant est l'acide sulfurique ou l'acide chlorhydrique.

4. Procédé de production selon l'une quelconque des revendications 1 à 3, dans lequel la réaction est réalisée à une température de 30 à 70°C.

5. Procédé de production selon l'une quelconque des revendications 1 à 4, dans lequel le solvant de réaction est uniquement constitué d'eau.

6. Procédé de production selon l'une quelconque des revendications 1 à 5, dans lequel le pH du mélange liquide lors de la charge se trouve dans un intervalle de 2,5 à 3,5 à 25°C.
